# EUROPEAN PATENT APPLICATION

(11) **EP 4 825 983 A1**
(43) Date of publication of application: **21.10.2026**
(21) Application number: 24904244.1
(22) Date of filing: 10.12.2024
(51) Int. Cl.: C12Q 1/6886

(54) **METHOD FOR PREDICTING PROGNOSIS OF BREAST CANCER BY USING IMMUNOSUPPRESSIVE FIBROBLAST ACTIVITY MEASUREMENT DATA**

(30) Priority: 11.12.2023 KR 20230178905; 05.12.2024 KR 20240179302
(71) Applicant: Korea Advanced Institute of Science and Technology, Daejeon 34141 (KR); National Cancer Center, Goyang-si, Gyeonggi-do 10408 (KR)
(72) Inventor: CHOI, Jung Kyoon, Daejeon 34141 (KR); KIM, Kyeong Hui, Daejeon 34141 (KR); JUNG, So Youn, Goyang-si, Gyeonggi-do 10382 (KR); KONG, Sun Young, Goyang-si, Gyeonggi-do 10415 (KR); PARK, Jong Bae, Seoul 06598 (KR)
(74) Representative: Mewburn Ellis LLP
(86) International application number: PCT/KR2024/020184
(87) International publication number: WO 2025/127679

(57) **Abstract**

The present disclosure relates to a method of providing information for predicting the prognosis of breast cancer using activity measurement data of immunosuppressive fibroblasts. The method of providing information for predicting the prognosis of breast cancer according to the present disclosure can provide various types of information not only for predicting the prognosis of breast cancer patients but also for further understanding the breast cancer tumor ecosystem.

## Description

### [Technical Field]

The present disclosure relates to a method of providing information for predicting the prognosis of breast cancer using activity measurement data of immunosuppressive fibroblasts, and more particularly, to a method of providing information for predicting the prognosis of breast cancer using a cell-state score of immunosuppressive fibroblasts, myofibroblasts, or both immunosuppressive fibroblasts and myofibroblasts, and a MYC target activity level.

### [Background Art]

Breast cancer is one of the most prevalent diseases, affecting nearly one in eight women in the United States. Furthermore, globally, breast cancer is the most frequently diagnosed cancer in women and is also a leading cause of cancer-related mortality among women. Over the past few decades, both the incidence and mortality rates of breast cancer have shown an increasing trend. Risk factors for breast cancer include various factors, including genetics, lifestyle, obesity, hormones, and age. Notably, the incidence of breast cancer is closely associated with age, and in South Korea, the highest incidence is reported among women aged 40 to 49.

Breast cancer is a heterogeneous disease characterized by significant variation between individual patients, referred to as inter-tumor heterogeneity, and even within each individual tumor, referred to as intra-tumor heterogeneity. Inter-tumor heterogeneity contributes to differences in disease stage and histopathological characteristics. These variations are reflected in subtype classification and provide important insights into prognosis and therapeutic approaches. Generally, breast cancer is classified into four major subtypes: luminal A, luminal B, HER2, and triple-negative breast cancer (TNBC).

To distinguish these breast cancer subtypes, immunohistochemistry (IHC) and prediction analysis of microarray 50 (PAM50) methodologies are commonly used. The IHC-based subtypes depend on the expression of estrogen and progesterone nuclear hormone receptors (ER/PR) and human epidermal growth factor receptor-2 (HER-2). Luminal A, the most prevalent subtype accounting for approximately 40% of cases, is characterized by being ER- and/or PR-positive while being HER-2-negative. Luminal B, which constitutes about 20% of cases, is characterized by being ER-, PR-, and HER-2-positive. In addition, HER2-positive breast cancer represents approximately 10% to 15% of cases and is ER- and PR-negative but HER-2-positive. Lastly, TNBC is characterized by being negative for ER, PR, and HER-2, accounting for about 15% to 20% of cases. On the other hand, the subtypes based on the PAM50 method are intrinsic subtypes of breast cancer determined by gene expression of 50 genes. PAM50-based subtypes offer improved risk prediction and disease management compared with IHC-based subtypes. In particular, the selection of therapy depends on the subtype. For example, luminal A responds better to endocrine therapy, whereas TNBC responds more favorably to chemotherapy. Therefore, accurate subtype classification is essential for selecting the most effective therapeutic strategy. Nevertheless, accurate classification of subtypes remains a challenge.

Breast cancer diagnosed at a younger age is associated with more aggressive characteristics and worse survival outcomes. Furthermore, young breast cancer (YBC) patients have a higher risk of metastasis and recurrence than old breast cancer (OBC) patients. However, the underlying mechanisms associated with age and tumor aggressiveness remain unknown. Moreover, the biological characteristics of cancer stem from complex interactions among numerous cellular components, including DNA, RNA, proteins, and small molecules. In particular, because the actual mediators and regulators of cellular signaling pathways in cancer are mostly observed at the protein level, it is necessary to extend beyond the transcriptional level to the translational level in order to elucidate the biological mechanisms of YBC. In conclusion, relying solely on genomic alterations poses significant difficulties due to the complexity of tumorigenesis processes, and a more comprehensive understanding of these mechanisms requires the integration of multiple layers of omics data, including genomic, transcriptomic, epigenomic, and proteomic domains.

In the present disclosure, multi-omics data were generated and analyzed for 289 Korean breast cancer samples, including 178 YBC and 111 OBC samples. The multi-omics data in the present disclosure include six data types ranging from genome, exome, and transcriptome sequencing to methylome, proteome, and phosphoproteome data. This integrated multi-omics study will provide novel and clinically relevant insights that may be difficult to capture when examining single-omics data alone.

### [Disclosure]

### [Technical Problem]

An object of the present disclosure is to provide a method of providing information for predicting the prognosis of breast cancer, the method comprising: obtaining a biological sample from a cancer patient; determining, from the obtained biological sample, a cell-state score of immunosuppressive fibroblasts, myofibroblasts, or both immunosuppressive fibroblasts and myofibroblasts; and predicting the prognosis of breast cancer based on the determined cell-state score.

Another object of the present disclosure is to provide a method of providing information for predicting the prognosis of breast cancer, the method comprising: obtaining multi-omics data from an isolated sample of a cancer patient; performing hierarchical clustering analysis based on the multi-omics data to classify each sample into a breast cancer subtype; and predicting a prognosis based on the classified subtype.

However, the technical problems to be solved by the present disclosure are not limited to the problems mentioned above, and other problems not mentioned will be clearly understood by those skilled in the art from the following description.

### [Technical Solution]

According to an embodiment of the present disclosure, a method of providing information for predicting the prognosis of breast cancer comprises: a) obtaining a biological sample from a cancer patient; b) determining, from the obtained biological sample, a cell-state score of immunosuppressive fibroblasts, myofibroblasts, or both immunosuppressive fibroblasts and myofibroblasts; and c) predicting the prognosis of breast cancer based on the determined cell-state score.

According to an embodiment of the present disclosure, the immunosuppressive fibroblasts may be characterized by at least one gene selected from the group consisting of *GEM, CXCL8, CXCL3, CXCL2, CXCL1, IL6, TNFAIP6, MT1A, THAP2, AKR1C1, FOSL1, CEBPB, IER3, LIF, SOD2, C11orf96, CD44, GPRC5A, KDM6B, TNFRSF12A, DDX21, NFKBIA, MAFF, UAP1, SLC3A2, WTAP, PPP1R15A, RND3, MYC, ADAMTS4, HSPD1, PTGS2, CREM, PLIN2, CYCS, BAG3, REL, NAMPT, CYTOR, ZC3H12A, BTG3, XBP1, HSPH1, HSPA9, EIF4A3, EIF5,* and *ERRFI1.*

According to an embodiment of the present disclosure, the immunosuppressive fibroblasts may be IL6+ inflammatory fibroblasts.

According to an embodiment of the present disclosure, the predicting of the prognosis of breast cancer using the cell-state score in step c) may be performed by comparing the cell-state score with a predetermined reference value.

According to an embodiment of the present disclosure, the prognosis may be evaluated as poor when the determined cell-state score of the immunosuppressive fibroblasts is higher than a median value.

According to an embodiment of the present disclosure, the prognosis may be evaluated as favorable when the determined cell-state score of the myofibroblasts is higher than a median value.

In addition, the present disclosure provides a method of providing information for predicting the prognosis of breast cancer, the method comprising: obtaining multi-omics data from an isolated sample of a cancer patient; performing hierarchical clustering analysis based on the multi-omics data to classify each sample into a breast cancer subtype; and predicting the prognosis of breast cancer based on the classified subtype.

According to an embodiment of the present disclosure, the multi-omics data may comprise at least one selected from the group consisting of genetic variations, tumor microenvironment components, immune cell fractions, immune signatures, fibroblast activity, TP53 mutation status, and MYC activity scores.

According to an embodiment of the present disclosure, the method of providing information for predicting the prognosis of breast cancer may further comprise, after the classification into the breast cancer subtype, comparing, for each breast cancer subtype, age groups, TP53 mutation status, and distributions of conventional breast cancer subtypes to identify heterogeneity of breast cancer subtypes among the clusters.

According to an embodiment of the present disclosure, the method of providing information for predicting the prognosis of breast cancer may further comprise, after the classification into the breast cancer subtype, analyzing tumor microenvironment (TME) and immune components for each breast cancer subtype to identify molecular characteristics of each breast cancer subtype.

According to an embodiment of the present disclosure, the method of providing information for predicting the prognosis of breast cancer may further comprise analyzing immune cell fractions by age for each breast cancer subtype to identify immune cell composition according to age.

According to an embodiment of the present disclosure, the breast cancer subtypes derived through the clustering may be classified into nine breast cancer subtypes.

### [Advantageous Effects]

A method of providing information for predicting the prognosis of breast cancer according to an embodiment of the present disclosure can provide information related to predicting the prognosis of a breast cancer patient.

A method of providing information for predicting the prognosis of breast cancer according to an embodiment of the present disclosure can enhance the understanding of the breast cancer tumor ecosystem, and in particular, can help elucidate underlying mechanisms for more aggressive characteristics observed in young-onset breast cancer (YBC).

In addition, multi-omics-based clustering analysis enables a systematic understanding of various breast cancer subtypes, based on which more accurate prognostic evaluation and personalized therapeutic strategies can be established.

However, the effects of the present disclosure are not limited to the above-mentioned effects, and should be understood to include all effects that can be inferred from the configuration of the disclosure described in the detailed description or the claims.

### [Description of Drawings]

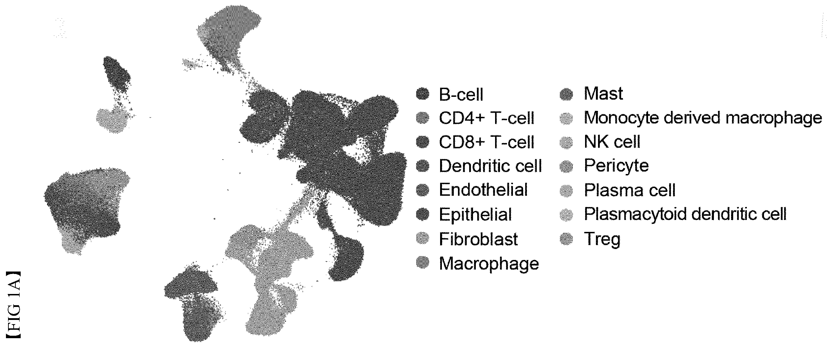

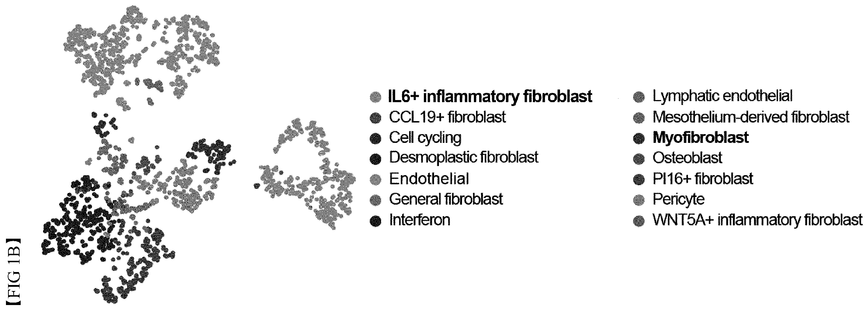

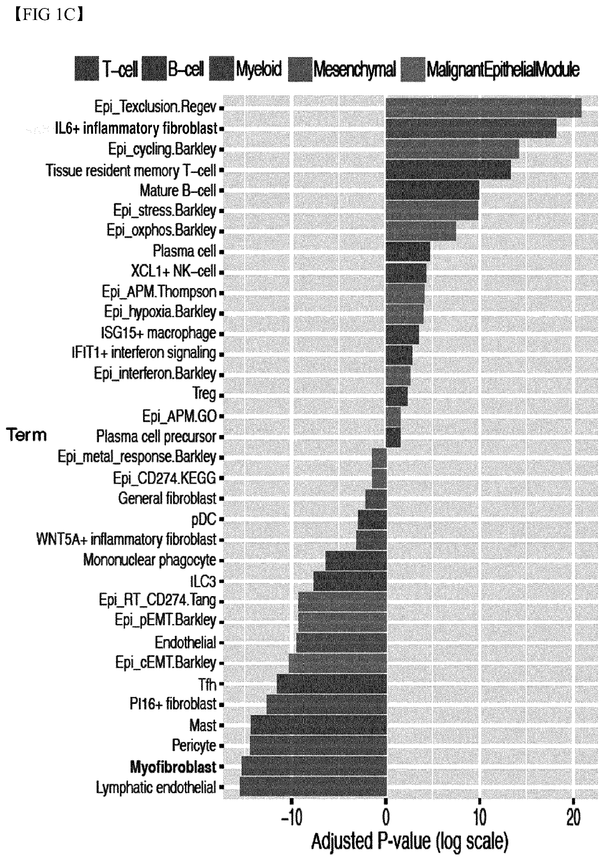

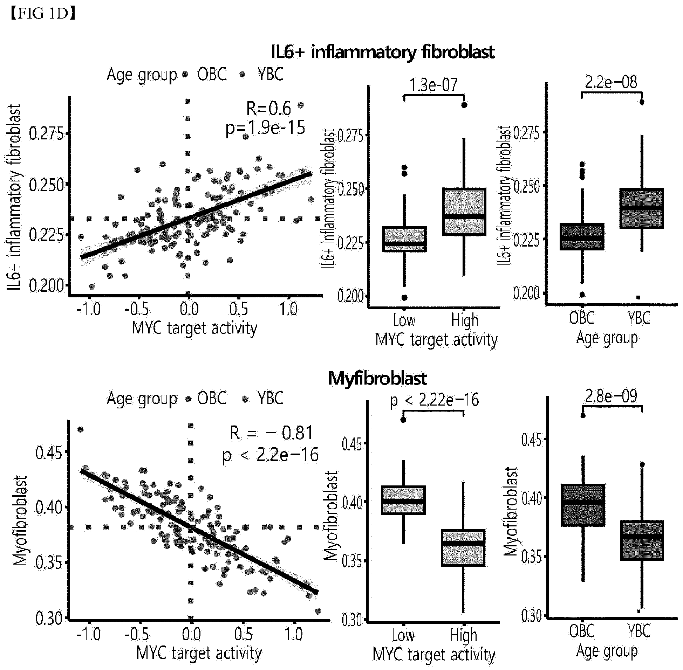

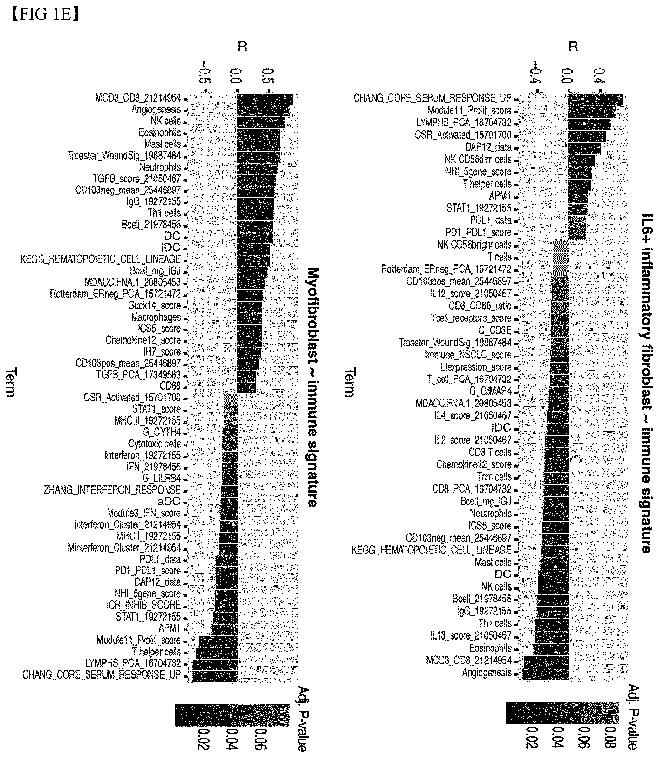

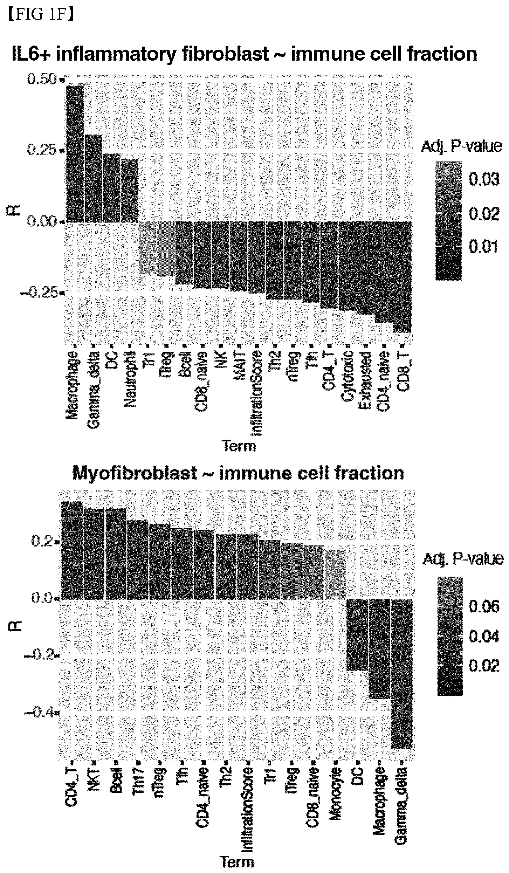

FIG. 1 relates to differences in the tumor microenvironment between tumors having high and low activity of immunosuppressive fibroblasts, and shows: (A) UMAP visualization of single-cell transcriptomes colored by cell type; (B) UMAP visualization of single-cell transcriptomes colored by mesenchymal cell state; (C) multiple comparisons of protein cell-state scores between MYC-high samples and MYC-low samples, wherein statistical analysis was performed using the Wilcoxon signed-rank test, and the false discovery rate (FDR) was controlled to be < 0.1; (D) results obtained by examining, through correlation analysis, effects of MYC target activity and age on protein scores of IL6+ inflammatory fibroblasts (upper panels) and myofibroblasts (lower panels) (left), comparison of cell-state scores between MYC-high tumors and MYC-low tumors (middle), and comparison of cell-state scores between YBC and OBC (right); (E) correlations between scores of IL6+ inflammatory fibroblasts (upper panels) and myofibroblasts (lower panels) and multiple immune scores; and (F) correlations between scores of IL6+ inflammatory fibroblasts (upper panels) and myofibroblasts (lower panels) and various immune cell fractions.

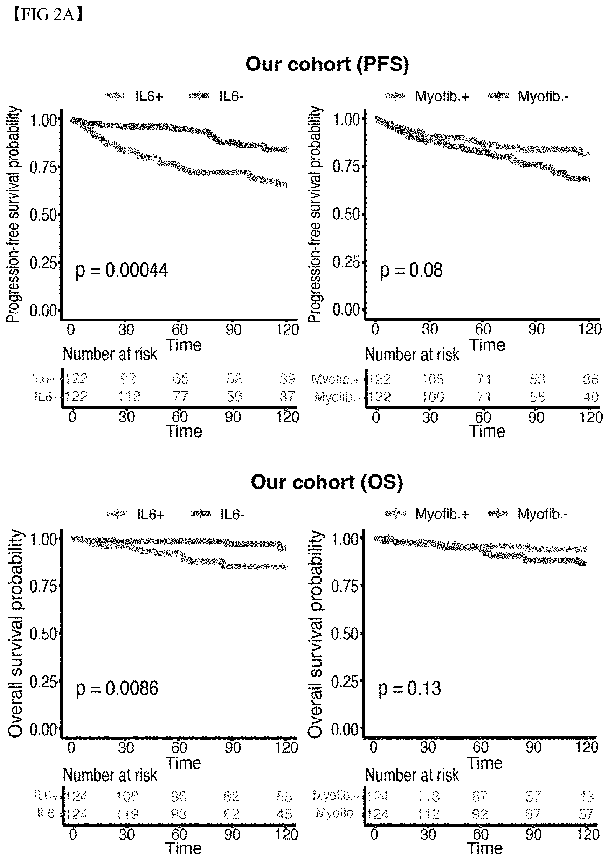

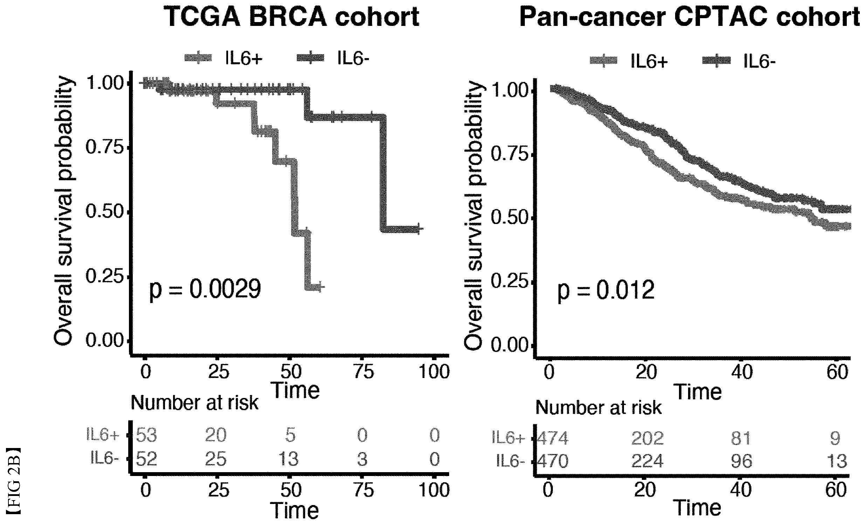

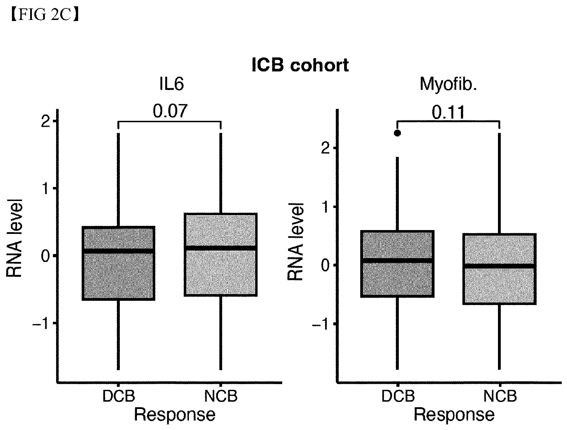

FIG. 2 relates to prognostic effects of IL6+ inflammatory fibroblasts and myofibroblasts, and shows evaluation of the scores of IL6+ inflammatory fibroblasts and myofibroblasts for predicting natural immune responses and clinical outcomes of immuno-oncology therapy, through: (A) survival analysis between high-score and low-score groups based on scores of IL6+ inflammatory fibroblasts (left) and myofibroblasts (right) in a breast cancer cohort, wherein Kaplan-Meier curves show progression-free survival (PFS) (upper panels) and overall survival (OS) (lower panels); (B) survival analysis between groups having high and low IL6+ inflammatory fibroblast scores in a breast cancer TCGA cohort (left) and a multi-cancer CPTAC cohort (right); and (C) comparison of scores of IL6+ inflammatory fibroblasts (left) and myofibroblasts (right) between durable clinical benefit (DCB) and no clinical benefit (NCB) groups in an immuno-oncology therapy cohort.

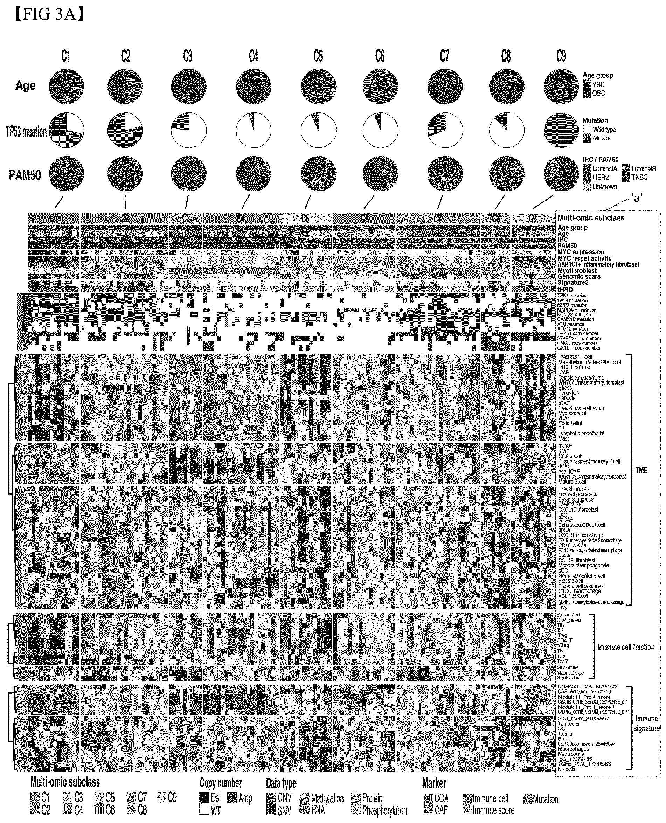

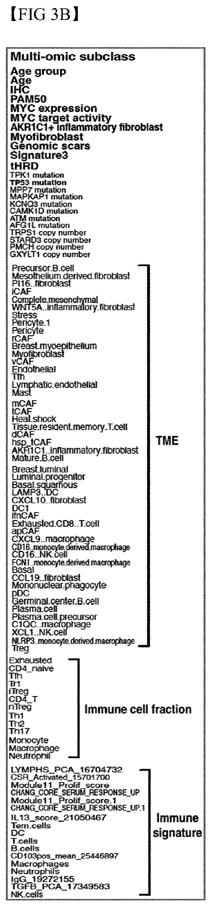

FIG. 3A shows newly performed unsupervised hierarchical clustering of breast cancer subtypes into nine detailed breast cancer subtypes using multi-omics data, wherein distributions of age groups, TP53 mutation status, and PAM50 subtypes for each multi-omics breast cancer subtype are indicated at the top, and sample information is shown together with levels of MYC expression, MYC target activity, IL6+ inflammatory fibroblasts, and myofibroblasts for each breast cancer subtype. At the bottom, markers normalized to z-scores are shown, including genetic variations, tumor microenvironment components, immune cell fractions, and immune scores including immune signatures. FIG. 3B is an enlarged view of the classification names indicated on the right side of FIG. 3A and marked as "a."

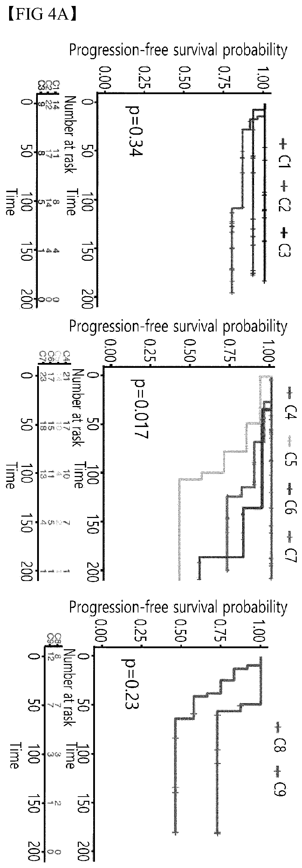

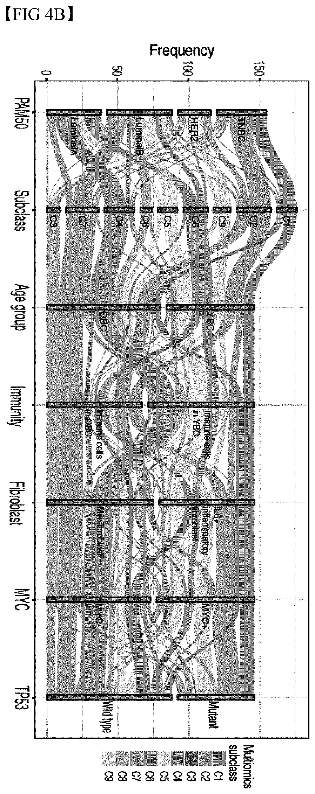

FIG. 4 relates to analysis of multi-omics clustering, and shows: (A) results of survival analysis for new subtypes using progression-free survival (PFS) within TNBC (left), luminal (middle), and HER2 (right) breast cancer subtypes; and (B) trends of markers, such as immune cell fractions, fibroblasts, MYC target activity, and TP53 mutation status, for each multi-omics breast cancer subtype.

### [Best Mode]

Hereinafter, embodiments will be described in detail with reference to the accompanying drawings. However, since various modifications may be made to embodiments, the scope of the present disclosure is not limited or restricted by these embodiments. It should be understood that all modifications, equivalents and substitutes for embodiments are included in the scope of the present disclosure.

The terms used in the embodiments are used for the purpose of description only, and should not be construed to be limited. A singular expression includes a plural expression unless the context clearly indicates otherwise. In the present specification, it should be understood that the term "comprising" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as an ideal meaning or excessively formal meanings unless clearly defined in the present application.

As used herein, the term "+" or "positive" may mean that a marker capable of characterizing a cell is expressed.

As used herein, the term "MYC" may refer to a proto-oncogene that lies at the intersection of various cell growth-promoting signaling pathways and, as a gene responsive to various ligand-membrane receptor complexes, regulates cell growth and proliferation.

In describing the embodiments, when a detailed description of related known technologies is determined to unnecessarily obscure the gist of the embodiments, the detailed description thereof will be omitted.

A method of providing information for predicting the prognosis of breast cancer according to the present disclosure comprises: a) obtaining a biological sample from a cancer patient; b) determining, from the obtained biological sample, a cell-state score of immunosuppressive fibroblasts, myofibroblasts, or both immunosuppressive fibroblasts and myofibroblasts; and c) predicting the prognosis of breast cancer based on the determined cell-state score.

In the present disclosure, the method of providing information for predicting the prognosis of breast cancer may identify various mesenchymal cell states of immunosuppressive fibroblasts, myofibroblasts, or both immunosuppressive fibroblasts and myofibroblasts through non-negative matrix factorization (NMF) analysis.

In the present disclosure, the immunosuppressive fibroblasts may be characterized by at least one gene selected from the group consisting of *GEM, CXCL8, CXCL3, CXCL2, CXCL1, IL6, TNFAIP6, MT1A, THAP2, AKR1C1, FOSL1, CEBPB, IER3, LIF, SOD2, C11orf96, CD44, GPRC5A, KDM6B, TNFRSF12A, DDX21, NFKBIA, MAFF, UAP1, SLC3A2, WTAP, PPPIR15A, RND3, MYC, ADAMTS4, HSPD1, PTGS2, CREM, PLIN2, CYCS, BAG3, REL, NAMPT, CYTOR, ZC3H12A, BTG3, XBP1, HSPH1, HSPA9, EIF4A3, EIF5,* and *ERRFI1.* More preferably, the immunosuppressive fibroblasts may be characterized by at least one gene selected from the group consisting of *AKR1C1, IL6, FOSL1, CXCL1, CXCL3,* and *CXCL8,* but are not limited thereto.

In the present disclosure, the immunosuppressive fibroblasts may be IL6+ inflammatory fibroblasts, and the IL6+ inflammatory fibroblasts may exhibit a strong positive correlation with MYC target activity and a strong negative correlation with patient age, respectively.

In addition, in the present disclosure, the IL6+ inflammatory fibroblast state may have negative correlations with macrophages, γδ T cells, and dendritic cells (DCs), and may have a positive correlation with neutrophils, in an analysis of correlations with immune cells.

In the present disclosure, the method of providing information for predicting the prognosis of breast cancer may evaluate scores by determining the activity of each cell state from a breast cancer sample and then distinguishing high and low scores based on the median value of each score in a cohort used for analysis.

In the present disclosure, the step of predicting the prognosis of breast cancer based on the determined cell-state score in step c) may be performed by comparing the cell-state score with a reference value, namely, the median value of each score in the cohort used for analysis. More specifically, the prognosis may be evaluated as poor when the determined cell-state score of the immunosuppressive fibroblasts is higher than a median value, and the prognosis may be evaluated as favorable when the determined cell-state score of the myofibroblasts is higher than a median value.

In the present disclosure, the method of providing information for predicting the prognosis of breast cancer may indicate immunotherapy resistance and a poor prognosis when the proportion of a particular cell state, more specifically IL6+ inflammatory fibroblasts, is high.

In addition, in the present disclosure, the prognosis of breast cancer may be evaluated using the determined level of IL6+ inflammatory fibroblasts and the determined level of myofibroblasts. In this case, the prognosis may be evaluated as poor when the determined activity of the immunosuppressive fibroblasts is higher than a median value, and the prognosis may be evaluated as favorable when the determined activity of the myofibroblasts is higher than a median value.

A method of providing information for predicting the prognosis of breast cancer according to the present disclosure comprises: obtaining multi-omics data from an isolated sample of a cancer patient; performing hierarchical clustering analysis based on the multi-omics data to classify each sample into a breast cancer subtype; and predicting the prognosis of breast cancer based on the classified breast cancer subtype.

In the present disclosure, the step of obtaining the multi-omics data may include collecting multi-omics data including genomic, transcriptomic, proteomic, and metabolomic data from a sample of a breast cancer patient. The multi-omics data may include genetic variations, tumor microenvironment components, immune cell fractions, immune signatures, fibroblast activity, TP53 mutation status, and MYC activity scores.

In the present disclosure, the method of providing information for predicting the prognosis of breast cancer may include integrating the collected multi-omics data and then analyzing the data using a hierarchical clustering technique to classify breast cancer subtypes. In this case, more detailed sub-classification of breast cancer may be enabled, and personalized treatment strategies may be developed through the detailed classification. More specifically, when breast cancer subtypes are classified using multi-omics clustering data according to the present disclosure, inter-tumor heterogeneity may be identified as samples are assigned to different breast cancer subtypes.

In the present disclosure, the breast cancer subtypes derived through the clustering may be classified into nine breast cancer subtypes, and the nine breast cancer subtypes may be referred to as C1 to C9.

In the present disclosure, the method of providing information for predicting the prognosis of breast cancer may further include, in order to evaluate concordance between multi-omics-based clustering results and conventional PAM50 or IHC subtypes and to identify inter-tumor heterogeneity, comparing age groups, TP53 mutation status, and distributions of conventional breast cancer subtypes for each breast cancer subtype to identify heterogeneity of breast cancer subtypes among clusters. In this case, although samples having the same PAM50 or IHC subtype generally belong to the same cluster, in some cases, the same subtype may be assigned to different breast cancer subtypes, thereby confirming inter-tumor heterogeneity.

In the present disclosure, the method of providing information for predicting the prognosis of breast cancer may further include analyzing genetic variations, tumor microenvironment (TME), and immune components in order to identify unique molecular characteristics of each breast cancer subtype. Through this analysis, the complex heterogeneity of breast cancer can be more deeply understood.

In the present disclosure, the method of providing information for predicting the prognosis of breast cancer may further include comparing immune cell fractions correlated with age for each multi-omics breast cancer subtype in order to identify differences in immune cell composition according to age and to understand the relationship between age and breast cancer subtypes.

### [Modes]

Hereinafter, preferred Examples are presented in order to assist the understanding of the present disclosure. However, the following Examples are just provided to more easily understand the present disclosure and the contents of the present disclosure are not limited by Examples.

### Example 1: Correlation Analysis Between MYC Activity and Immune Cells in the Tumor Ecosystem of Breast Cancer

To systematically characterize the tumor ecosystem of breast cancer associated with MYC activity, single-cell transcriptomes of 450,877 cells obtained from 106 tumor samples and 28 normal samples were analyzed to define various cell states. Specifically, non-negative matrix factorization (NMF) analysis was applied to deconvolute gene expression into multiple cell states characterized by up to 50 marker genes for each cell type. For example, the analysis identified various mesenchymal cell states, including IL6+ inflammatory fibroblasts, CCL19+ fibroblasts, and myofibroblasts. Based on the protein levels of the markers, a cell-state score was determined for each breast cancer sample. Meanwhile, the IL6+ inflammatory fibroblasts may be identified by the expression of marker genes including AKR1C1, IL6, FOSL1, and CXCL1/3/8.

Next, correlations were determined between the cell-state scores and MYC target activity or the age at diagnosis across the cohort samples. Consistently with respect to MYC target activity and patient age, the IL6+ inflammatory fibroblast state and the myofibroblast state exhibited strong positive and negative correlations, respectively (FIGS. 1C and 1D). The IL6+ inflammatory fibroblasts were identified by marker genes including AKR1C1, IL6, FOSL1, and CXCL1/3/8. AKR1C1, which encodes a member of the aldo-keto reductase superfamily, regulates the metabolism of hormones such as estrogen and progesterone. In fact, this fibroblast state was overrepresented in organs associated with reproductive hormones, including the breast, uterus, fallopian tubes, ovaries, and prostate. In endometrial cancer, estrogen and progesterone levels are regulated by AKR1C1 and AKR1C3. MYC protein synthesis is influenced by post-transcriptional regulation involving the internal ribosome entry site in the 5'-untranslated region. In particular, *IL6,* one of the marker genes for the IL6+ inflammatory fibroblast state, has been reported to enhance MYC translation through this mechanism. This translational control of MYC by IL6 has been proposed as a pathway linking inflammation and cancer. Therefore, it is suggested that IL6 released from IL6+ inflammatory fibroblasts could potentially contribute to increased MYC signaling in connection with hormone metabolism in young breast cancer patients.

Unlike young breast cancer (YBC) samples in which MYC targets are enriched, older breast cancer (OBC) samples exhibit a more enriched myofibroblast state. The negative correlation of the myofibroblast state with MYC target activity and patient age is consistent with this finding. Notably, cell-state scores determined using transcriptomes instead of proteome data showed a reduced correlation of MYC target activity with IL6+ inflammatory fibroblasts and myofibroblasts. Accordingly, the importance of measuring protein activity was confirmed in understanding the molecular dynamics of breast cancer, and utilizing proteomic data allowed for a more accurate characterization of the tumor ecosystem.

Next, the correlation between the two fibroblast states identified across the breast cancer samples and the immune scores as well as immune cell fractions was investigated. As a result, both immune activity parameters showed an overall negative correlation with the IL6+ inflammatory fibroblast score, but a positive correlation with the myofibroblast score (FIGS. 1E and 1F). More specifically, various types of T cells exhibited a negative correlation with the IL6+ inflammatory fibroblast state, unlike macrophages, γδ T cells, and dendritic cells (DCs) (FIG. 1F). Macrophages are known to promote tumorigenesis of breast cancer through processes such as angiogenesis, immunosuppression, tumor invasion, and metastasis. Tumor-infiltrating γδ T cells and DCs have been identified as predictors of unfavorable survival outcomes in breast cancer. Macrophages, γδ T cells, and DCs were identified as three immune cell types inversely related to the myofibroblast state (FIG. 1F). Neutrophils exhibited a positive correlation with the IL6+ inflammatory fibroblast state (FIG. 1F). CXCL1/2/3, which are markers of the IL6+ inflammatory fibroblast state, are known to recruit neutrophils. These genes may contribute to poor prognosis and resistance to immunotherapy in breast cancer by protecting tumor cells from cytotoxic T cells.

Meanwhile, as an evaluation criterion for the scores of the IL6+ inflammatory fibroblasts and myofibroblasts, high and low scores were distinguished based on the median value of each score in the cohort used for the analysis. In one embodiment, the prognosis of breast cancer was evaluated as a poor prognosis when the cell-state score of the IL6+ inflammatory fibroblasts was greater than the median value, and the prognosis was evaluated as a favorable prognosis when the cell-state score of the myofibroblasts was greater than the median value.

In conclusion, these findings suggest that IL6+ inflammatory fibroblasts may contribute to T-cell exclusion while attracting pro-tumorigenic immune cells such as macrophages, γδ T cells, DCs, and neutrophils. The activity of MYC target genes exhibited a similar correlation pattern with the immune cell fractions, particularly in terms of T-cell repulsion, which is consistent with the T-cell exclusion signature observed in the MYC-active tumors (FIG. 1C). That is, it was confirmed that IL6+ inflammatory fibroblasts can play a role in creating a pro-inflammatory, immunosuppressive tumor microenvironment in MYC-active breast cancer.

### Example 2: Clinical Significance of Age-Related Molecular Characteristics

The prognostic potential of age-related characteristics was evaluated in terms of progression-free survival (PFS) and overall survival (OS) data of the cohort. First, all samples were divided into high and low groups based on the median value of each characteristic. In the two fibroblast states, the IL6+ inflammatory fibroblasts were associated with a worse survival rate, whereas the myofibroblasts correlated with a better survival rate (FIG. 2A). This could be partially explained by their relationship with the immune microenvironment (FIGS. 1D and 1E). Taken together, markers identified to be associated with the age at diagnosis were shown to significantly affect survival outcomes. This also confirmed that overall survival (OS) was lower in the group with a high IL6+ inflammatory fibroblast score in the breast cancer TCGA cohort and the multi-cancer CPTAC cohort (FIG. 2B).

To further investigate the immunological aspects of the two fibroblast states, transcriptome data from different cohorts across various cancer types treated with immune checkpoint blockade (ICB) were collected. After classifying the samples into durable clinical benefit (DCB) or no clinical benefit (NCB) according to the response, the predictive power of the two fibroblast states regarding ICB response was evaluated. As a result, the IL6+ inflammatory fibroblast state was more common in the NCB group, whereas the myofibroblast state was more prevalent in the DCB group (FIG. 2C). These findings suggest that the fibroblast states act as crucial regulators of the immune microenvironment and can account for both the natural immune response and the response to ICB treatment. As the predictive power of these markers has been demonstrated in various cancers, they are highly likely to be utilized as biomarkers capable of guiding immunotherapy strategies.

### Example 3: Breast Cancer Subtype Classification Using Multi-Omics Data

To investigate whether multi-omics data can more precisely classify breast cancer into subtypes, hierarchical clustering was performed using 6,000 multi-omics features from 142 samples for which all data types were available. As a result, nine distinct breast cancer subtypes (C1 to C9) were identified (FIG. 3). Analysis of the distribution of age groups, TP53 mutations, and PAM50 subtypes revealed that samples with the same PAM50 or IHC subtype generally belonged to the same cluster. However, in some cases, the same subtype was assigned to different subtypes in the multi-omics clustering, confirming inter-tumor heterogeneity. Specifically, triple-negative breast cancer (TNBC) samples were mainly concentrated in C1, C2, and C3. However, C1 and C2 mostly consisted of YBC samples harboring TP53 mutations, whereas C3 was predominantly composed of TP53 wild-type OBC samples. In addition, subtypes enriched with Luminal A/B showed a high proportion of TP53 wild-type samples. Particularly, OBC samples were heavily included in C4, C7, and C8, whereas YBC samples were prominent in C5 and C6. C9 consisted exclusively of TP53 mutant samples and was mostly classified as the HER2 subtype according to the PAM50 criteria. These results demonstrate the complex molecular diversity of breast cancer and highlight that multi-omics-based clustering enables a deeper understanding of breast cancer heterogeneity. This approach can provide new insights into subclassifying breast cancer and developing tailored therapeutic strategies. It is expected that multi-omics clustering will improve the predictive power for breast cancer severity and guide more personalized treatment strategies.

Furthermore, to comprehensively understand the molecular characteristics of each subtype, various markers including genetic alterations, tumor microenvironment (TME) components, immune cell fractions, and immune signature scores were analyzed. To explore functional differences, the markers were clustered according to their expression patterns, thereby confirming unique molecular characteristics for each multi-omics subtype. Specifically, as for the TME markers, myofibroblasts and vascular CAFs (vCAFs) formed an independent cluster, showing high levels in C3, C4, and C7, while showing low levels in C1, C5, and C9. Conversely, IL6+ inflammatory fibroblasts clustered together with tumor-like CAFs (tCAFs), matrix CAFs (mCAFs), and dividing CAFs (dCAFs), and were mainly expressed in C1 and C9. These clusters are groups predominantly composed of TP53-mutant YBC, and their expression was low in C3 and C4.

To validate the differences in immune cell composition according to age, the immune cell fractions correlated with age (FIG. 1F) were compared for each multi-omics subtype. In the clustering analysis results, immune cells enriched in YBC (macrophages, neutrophils, and monocytes) were grouped into a single cluster, and immune cells enriched in OBC formed two independent clusters composed of CD4+ T cells and helper T cells, respectively. In addition, in the TNBC-enriched subtypes (C1-C3), the fraction of helper T cells, which play an important role in promoting anti-tumor responses, was low, suggesting the potential to promote immune evasion in TNBC tumors.

Nevertheless, significant heterogeneity was observed even within these TNBC-enriched subtypes. For example, C3 exhibited the highest levels of myofibroblasts and vCAFs, suggesting a relatively stable and less aggressive nature, showing different characteristics despite being classified as TNBC. Furthermore, while CD4+ T cells were abundantly observed in C2, C3, and C7, immune cells high in YBC were low in these subtypes, suggesting that the active immune activity of C2 and C3 could induce a more potent anti-tumor response than C1. In addition, it was confirmed that immune-related cell state markers and interferon CAFs (ifnCAFs) were highly expressed in C2 and C3. These subtypes showed high immune signature scores, suggesting active immune responses such as strong lymphocyte infiltration. These results support the robust immune activity characterizing these subtypes.

That is, through the multi-omics clustering analysis according to the present disclosure, the complex heterogeneity of breast cancer in the tumor microenvironment and immune environment was confirmed. Their complex interactions characterize breast cancer subtypes, and the importance of molecular profiling to capture this complexity was confirmed. These research findings indicate that a multi-omics approach is an essential element in cancer research, through which dynamic changes in tumors can be more precisely understood, and more effective personalized treatment strategies can be developed.

### Example 4: Clinical Prognosis Evaluation Using Multi-Omics Subtypes

To confirm the clinical implications of the multi-omics subtypes of Example 3, the clinical prognosis of each subtype was evaluated, and the results are shown in FIG. 4A. As a result of the evaluation, TNBC is generally associated with a poor prognosis, but TNBC tumors belonging to C3 showed a favorable prognosis, reflecting non-aggressive molecular characteristics (left). In the Luminal A/B-enriched subtypes (C4 to C7), significant differences in survival outcomes were observed. In particular, C5, the most malignant Luminal A/B subtype, showed the worst survival rate, while C4, the most non-malignant, exhibited the best survival rate (middle). In addition, the prognosis of C9 was worse than that of C8, which is interpreted to be due to the high MYC score and low myofibroblast score in C9 (right).

Furthermore, the molecular characteristics of each breast cancer subtype were summarized by integrally visualizing the distribution of key markers for each subtype, including age groups and PAM50 subtypes (FIG. 4B). This analysis effectively captured the complex heterogeneity within the breast cancer samples, as it included key factors such as age-related immune cell fractions, fibroblast states, MYC target activity, and TP53 mutation status. These results provide crucial insights into the unique molecular characteristics defining each subtype and the interactions among age, immune cell composition, fibroblast activity, and key oncogenic pathways such as MYC. In particular, a strong correlation was observed between age groups and fibroblast states, suggesting that this interaction could potentially affect tumor aggressiveness and clinical outcomes. In conclusion, the multi-omics approach according to the present disclosure enables precise subclassification of breast cancer and allows for an understanding of the molecular heterogeneity driving disease progression, which can provide guidelines for more accurate prognosis evaluation and the development of personalized treatment strategies.

As described above, although the embodiments have been described by the restricted drawings, various technical modifications and variations can be applied on the basis of the embodiments by those skilled in the art. For example, even if the described techniques are performed in a different order from the described method, and/or components described above are coupled or combined in a different form from the described method, or replaced or substituted by other components or equivalents, an appropriate result can be achieved.

Therefore, other implementations, other embodiments, and equivalents to the appended claims fall within the scope of the claims to be described below.

## Claims

1. A method of providing information for predicting the prognosis of breast cancer, the method comprising:
a) obtaining a biological sample from a cancer patient;
b) determining, from the obtained biological sample, a cell-state score of immunosuppressive fibroblasts, myofibroblasts, or both immunosuppressive fibroblasts and myofibroblasts; and
c) predicting the prognosis of breast cancer based on the determined cell-state score.

2. The method of claim 1, wherein the immunosuppressive fibroblasts are **characterized by** at least one gene selected from the group consisting of *GEM, CXCL8, CXCL3, CXCL2, CXCL1, IL6, TNFAIP6, MT1A, THAP2, AKR1C1, FOSL1, CEBPB, IER3, LIF, SOD2, C11orf96, CD44, GPRC5A, KDM6B, TNFRSF12A, DDX21, NFKBIA, MAFF, UAP1, SLC3A2, WTAP, PPP1R15A, RND3, MYC, ADAMTS4, HSPD1, PTGS2, CREM, PLIN2, CYCS, BAG3, REL, NAMPT, CYTOR, ZC3H12A, BTG3, XBP1, HSPH1, HSPA9, EIF4A3, EIF5,* and *ERRFI1.*

3. The method of claim 1, wherein the immunosuppressive fibroblasts are IL6+ inflammatory fibroblasts.

4. The method of claim 1, wherein the predicting of the prognosis of breast cancer using the cell-state score in step c) is performed by comparing the cell-state score with a predetermined reference value.

5. The method of claim 1, wherein the prognosis is evaluated as poor when the determined cell-state score of the immunosuppressive fibroblasts is higher than a median value.

6. The method of claim 1, wherein the prognosis is evaluated as favorable when the determined cell-state score of the myofibroblasts is higher than a median value.

7. A method of providing information for predicting the prognosis of breast cancer, the method comprising:
1) obtaining multi-omics data from an isolated sample of a cancer patient;
2) performing hierarchical clustering analysis based on the multi-omics data to classify each sample into a breast cancer subtype; and
3) predicting the prognosis of breast cancer based on the classified subtypes.

8. The method of claim 7, wherein the multi-omics data include at least one selected from the group consisting of genetic variations, tumor microenvironment components, immune cell fractions, immune signatures, fibroblast activity, TP53 mutation status, and MYC activity scores.

9. The method of claim 7, further comprising, after the classification into the breast cancer subtype in step 3), comparing, for each subtype, age groups, TP53 mutation status, and distributions of conventional breast cancer subtypes to identify heterogeneity of breast cancer subtypes among the clusters.

10. The method of claim 7, further comprising, after the classification into the breast cancer subtype in step 3), analyzing tumor microenvironment (TME) and immune components for each subtype to identify molecular characteristics of each subtype.

11. The method of claim 7, further comprising, after the classification into the breast cancer subtype in step 3), analyzing immune cell fractions by age for each subtype to identify immune cell composition according to age.

12. The method of claim 7, wherein the breast cancer subtypes derived through the clustering are classified into nine subtypes.
